# EUROPEAN PATENT APPLICATION

(11) **EP 1 690 504 A1**
(43) Date of publication of application: **16.08.2006**
(21) Application number: 06250737.1
(22) Date of filing: 10.02.2006
(51) Int. Cl.: A61B 17/32, B24B 3/40

(54) **Arthroscopic shaver and method of manufacturing same**

(30) Priority: 11.02.2005 US 651646 P
(71) Applicant: Arthrex, Inc., Naples, Florida 34108-1945 (US)
(72) Inventor: Hacker, Randall L., Naples 34117-4223 Florida (US); Van Wyk, Robert A., Largo 33777 Florida (US)
(74) Representative: Collins, John David

(57) **Abstract**

An arthroscopic shaver with an inner cutting window (110) preferably having a plurality of teeth positioned along the lateral cutting edges, the teeth being configured for easy penetration into tissue to prevent ejection of tissue from the cutting window during closure. The inner cutting edges have acute included angles on the first advanced lateral edge (113) ad the currilinear distal edge (119) and are formed in a milling operation using a milling cutter having an end radius equal to that of the surfaces forming the inner surfaces of the cutting edges. The teeth may be symmetrically or asymmetrically placed about the tube axis when viewed in a plan view.

## Description

### FIELD OF THE INVENTION

The present invention relates to arthroscopic surgery and, more particularly, to a shaver blade for arthroscopic surgery.

### BACKGROUND OF THE INVENTION

Resection of tissue by an arthroscopic shaver blade is accomplished by cooperative interaction between the edges of the inner and outer cutting windows. As the inner and outer windows come into alignment, tissue is drawn into the opening formed by these windows by a vacuum applied to the shaver inner lumen by an external vacuum source. Continued rotation of the inner member causes the inner cutting edges to approach the outer cutting edges. Tissue in the cutting window between the inner and outer edges is either trapped between the edges or ejected from the window. Tissue trapped between the edges is either cut by the edges as they approach each other, or torn by the cutting edges as they pass and rotate away from each other. The resected tissue is aspirated from the site through the inner lumen of the inner tube.

Resection efficiency is improved by decreasing the relative portion of the material that is ejected from the window, and increasing the portion that is trapped between the edges and resected. Decreasing the relative portion ejected from the window is accomplished by increasing the sharpness of the cutting edges and adding teeth to either the inner cutting edges or outer cutting edges or both. Increasing the sharpness is accomplished by decreasing the included angle of a cutting edge by decreasing the edge radius, and/or by decreasing the roughness of the surfaces over which tissue must slide during resection. Shavers having inner cutting edges with teeth are well known in the art. U.S. Patent No. 5,217,479 to Shuler and U.S. Patent No. 5,269,798 to Winkler describe shavers having inner cutting edges with teeth which are formed by a "through-cutting" process such as wire Electrical D*i*scharge Machining (wire EDM) or by grinding. The so-formed teeth are efficient at retaining tissue within the window so that it can be cut by the low included angle outer cutting edges as the inner and outer edges converge. The inner cutting edges do little cutting since the teeth form a very large included angle cutting edge. The Cuda™ by Linvatec Corporation (Largo, Florida) and the Tomcat™ by Stryker Incorporated (Kalamazoo, Michigan) have teeth on both the inner and outer cutting edges, the edges being formed by a two-dimensional, through-cutting process such as grinding or wire EDM. The edges formed have large included angles, geometry inefficient for cutting tissue. Shavers having these two-dimensionally shaped teeth on the inner and outer cutting edges separate tissue principally by tearing, as the edges pass each other during closure of the cutting window. Such tearing is undesirable since the torn tissue may frequently become wrapped into the gap between the inner and outer tubes so as to prevent the tissue from aspirating from the site thereby clogging the instrument.

Van Wyk et al., in U.S. Patent No. Patent 6,053,928, describe a shaver having a plurality of teeth on the laterally opposed cutting edges of an outer window, the cutting edges being symmetrical when viewed in a plane perpendicular to the axis of the tube. The cutting edges are formed so that, when viewed in any such plane, the edges have low included angles in the valleys between the teeth as well as the teeth. The Great White™ shaver by Linvatec, constructed in accordance with the principles of the Van Wyk patent, is very efficient at removing tissue and experiences reduced clogging due to the sharpness of the outer cutting edges which prevents tissue wrapping in the gap between the tubes.

Improvements in the tissue removing efficiency of shaver blades have been accomplished primarily through improvements in the design and manufacturing of the outer cutting edges. It is much easier to produce advanced geometries on an outer cutting window than on an inner cutting window because edges on the outer window can be produced by grinding. Multi-axis Computer Numerically Controlled (CNC) grinding machines and grinding wheels with shaped peripheries are able to make a myriad of edge configurations. Conversely, it is only possible to grind inner cutting edges with large included-angles. These ground edges may have teeth of various sizes and shapes, but little other geometries are possible. And such teeth are quite efficient for preventing tissue from being ejected as the window closes.

Advanced geometry outer cutting edges have little effect on the efficiency of a shaver when cutting bone. Bone is resected not by cooperative interaction of the inner and outer cutting edges, but by the inner cutting edges only. Accordingly, the geometry of the inner cutting edges has a much greater effect on the shaver performance when cutting bone.

The process of cutting of bone by a cutting edge has two phases: initiation and propagation. Initiation of the cut refers to the penetration of the bone by the cutting edge. For resection of bone to occur, the cutting edge must penetrate the bone rather than bounce off. For this to occur, the compressive stress (force divided by area) generated at the cutting edge/bone interface must exceed the compressive strength of the bone. The force applied by the cutting edge is determined by the handpiece and operator. The applied stress at the cutting edge can be increased to the required level by decreasing the area over which the force is applied, that is, by "sharpening" the cutting edge. An axe head has two sides: a wedge-shaped side for cutting, and a blunt side for pounding. Both sides apply the same force. The compressive stress applied to a log is much higher on the wedge-shaped side since the area of the edge is much less. This higher pressure causes localized "failure" in the log thereby allowing the cutting edge to penetrate the log. In the same manner, it is necessary to minimize the area of the cutting edge on a shaver inner blade in order to initiate a cut in bone. This can be accomplished by making knife-like edges, two-dimensional teeth, or pyramid shaped teeth. All will cause localized failure in the bone which they encounter and will penetrate the bone.

The second phase, propagation, is accomplished after initial penetration occurs. During propagation, the cutting edge advances further into the bone with spreading of the bone by the cutting edges causing a tensile failure in the material ahead of the edge. A "crack" is propagated ahead of the cutting edge. The direction of propagation is generally at a shallow angle with the surface of the bone. Accordingly, a "chip" of removed bone forms and slides along the surface of the cutting edge away from the edge. The propagation continues until the crack intersects a free surface so that a piece of material is removed, or localized fracturing of the chip occurs.

While a variety of cutting edge configurations can cause initiation, propagation requires a wedge-shaped edge decreasing in width in the rotation direction and with an apex lying in a plane approximately parallel to the free surface of the bone. Decreasing the included angle of the edge increases both the ease or initiation and the ease or propagation.

Several currently available shavers have wedge-shaped inner cutting edges. Among these are the Full Radius Resector by Linvatec Corporation (Largo, Florida), the Resector Full Radius by Stryker Corporation (Kalamazoo, Michigan), and the Full Radius Blade by Smith and Nephew (Andover, Massachusetts). These prior art shavers have low included-angle inner cutting edges formed by an axial channel cut into the distal end of the shaver, the channel width being approximately 70% of the inner tube diameter. The channel intersects the tube outer surface so as to form more or less wedge-shaped (knife-like) cutting edges from the proximal end of the window to the tangency of the distal radius. The edges formed are quite efficient for bone cutting. In the distal radius, however, the geometry is not well suited to bone cutting. The intersection of the channel with the spherical outer surface creates a transition from low included-angle cutting edges to high-included angle edges. This limits the relative angles between the surface of the bone to be resected and the axis of the shaver at which the shaver will cut effectively. When the angle between the axis of the shaver and the bone surface is low and cutting is done primarily with the more proximal knife-like portion of the edges, the shavers cut efficiently. However, when the angle between the axis and the bone surface is high, so that the distal radius of the shaver is brought into contact with bone, the high included angle portion of the edge does not penetrate the bone and causes the shaver to bounce away from the bone.

Heisler et al., in U.S. Patent No. 6,001,116, teaches a shaver inner cutting member with low included angle cutting edges which extend through the distal radius. The inner member, produced by wire EDM, has a "through cut" cutting window formed by two laterally opposed fingers which extend distally from the distal end of the inner tube to form two cutting windows. The resilient cutting edges so formed are able to deflect inward when subjected to high cutting forces, so as to increase the clearance between the cutting edges when cutting bone, for example. The geometry of the cutting edges, however, requires that they be produced by wire EDM. The edges produced are irregular and have rough surface finishes on the surfaces over which tissue must slide during the cutting process and this limits the efficiency of the shavers. The edge geometry is well suited for effective cutting to occur in the distal radius thereby making the shaver efficient at relatively large angles to the bone surface and an effective end cutter.

Decreasing the included angle of a cutting edge to improve cut initiation in bone requires that the material of which the edge is made have a suitably high yield strength. The cutting edge is subjected to a compressive stress equal to that applied to the bone undergoing resection. Insufficient yield strength of the cutting edge material results in plastic deformation of the cutting edges. This "mushrooming," in turn, results in dulling of the cutting edge and the generation of metallic debris as the deformed inner cutting edges interfere with the outer cutting edges and as the deformed metal causes galling of the inner surface of the outer tube in the region of the cutting window. Some shavers are made with the distal-most portion of the inner tube machined from a gall-resistant alloy such as Nitronic 60 or Gall-Tough to prevent galling of their distal bearing surfaces. These alloys generally have low yield strengths, not well suited to cutting edges for resection of bone. Other manufacturers produce their entire inner tube from an easily machined 300 series stainless steel, the distal portion being coated with a gall-resistant material. However, these 300 series stainless materials generally have low yield strengths, particularly in shavers in which the inner tube is formed from a single piece of tubing. This single-piece construction requires that the end of the tube be closed by plastic deformation. Closing of the tube in this manner requires a material which can undergo significant deformation without fracturing - a material with a low yield strength. Shavers made with 300 series inner cutting edges are not well suited to resection of bone since the edges undergo plastic deformation, unless the included angle of the cutting edges is increased to decrease the compressive stress at the edge.

In view of the above, a shaver well suited to efficient resection of all tissue types including bone requires that the inner cutting edges have a low included angle and that the materials from the edges have a high yield strength. Producing such cutting edges, however, is problematic since, as noted previously, the geometry of inner cutting edges does not allow their manufacture by grinding. The inner cutting edges of currently available shavers with wedge-shaped inner cutting edges are produced by EDM, a spark-erosion process which uses a shaped electrode with a complementary form to produce contours on a partpiece. In EDM, pulses of high voltage are applied between the shaped electrode and the partpiece, each pulse producing an arc which vaporizes workpiece and electrode material, the vaporized material being flushed away by the dielectric fluid in which the process occurs. The surface finish produced on the partpiece by the process is a series of overlapping craters made by the arc, the roughness being determined by the parameters used in the process. The surface is also covered by resolidified metal (recast) which was not carried away by the flow of dielectric fluid during the forming process. This recast material is extremely brittle due to its rapid solidification rates, has cracks in it, and has portions which may be only loosely bonded to the surface of the partpiece. The EDM process is poorly suited to the manufacture of cutting edges, particularly those for use on tissue. The surfaces produced are rough, so as to cause tissue to embed in the surface rather than slide over it. The edges are irregular and have rounded portions due to localized melting and resolidification of the edge material.

Milling is able to produce channels like those used in the prior art shavers currently available, however, the small size of the channels makes the milling of these channels problematic. A shaver with a 4 millimeter outer tube diameter will have an inner tube diameter of approximately 3.3 millimeters. The channel in a shaver of this size with parallel wedge-shaped cutting edges will be approximately 2 millimeters wide. Milling a channel of this width requires the use of an end-mill with a diameter of about 1.5 millimeters to allow roughing of the channel followed by finishing passes. An end-mill of this diameter is extremely fragile and requires high rotational speeds and low feed rates, both for preservation of the end-mill and to prevent the end-mill from flexing (wandering) and making products with a high degree of dimensional variability. Producing such a channel by milling with an end-mill is not economically feasible. This is especially true when the distal portion of the inner member is made from the high yield-strength required for a good bone cutter.

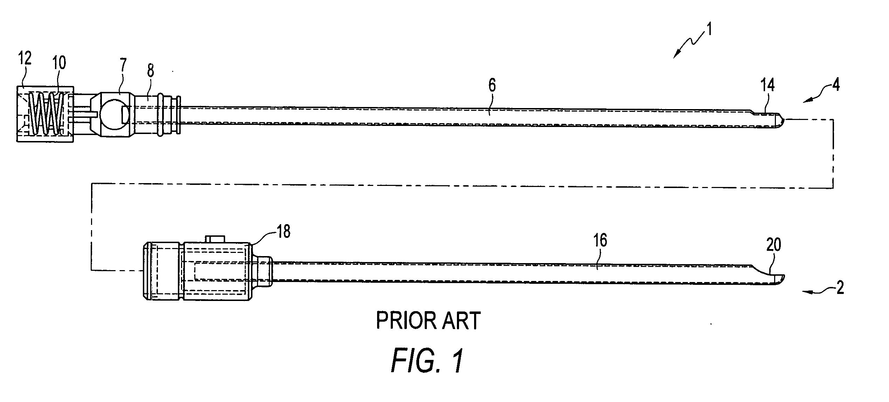

Referring to Figure 1, a prior art shaver 1 has an outer assembly 2 and an inner assembly 4 which is rotatably positioned therein. Inner assembly 4 has an elongated distal tubular portion 6, and a proximal hub assembly 7 having an inner hub 8, a spring 10, and a spring retainer 12. Hub assembly 8 is configured to transmit rotary motion from a powered handpiece to the inner assembly 4. Inner tubular portion 6 has a distal end 14 which forms a cutting window. Outer assembly 2 has an elongated distal tubular portion 16 with a distal end 20 forming a cutting window, and a proximal hub assembly 18 suitable for removably mounting in a powered handpiece.

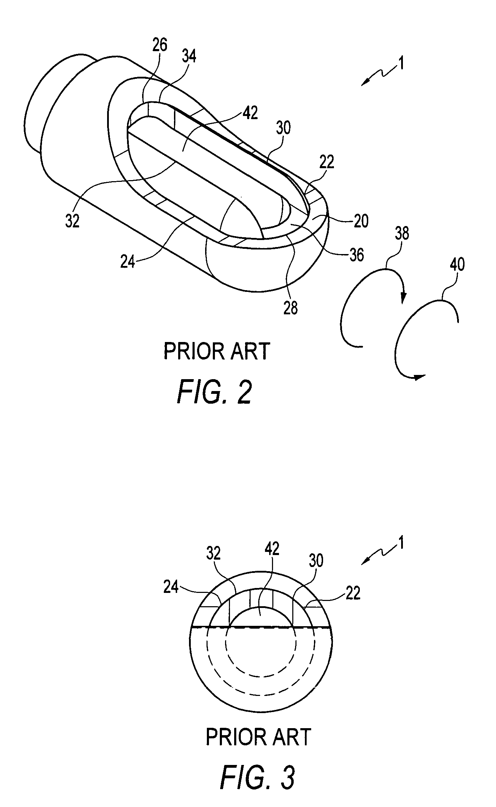

As seen in Figures 2 and 3 showing the distal end of prior art shaver 1, outer cutting window 20 has a first lateral cutting edge 22 and a second lateral cutting edge 24 connected by a curvilinear proximal edge 26 and a curvilinear distal edge 28. Inner cutting window 14 has a first lateral cutting edge 30, a second lateral cutting edge 32, a proximal curvilinear edge 34, and a distal portion 36. Shaver 1 is generally operated in an oscillate mode when cutting soft tissue. That is, inner assembly 4 is rotated within outer assembly 2 a predetermined number of revolutions in a first direction 38, and then rotated a predetermined number of revolutions in a second, opposite direction 40. The action is then repeated. When inner assembly 4 is rotated within outer assembly 2 in first direction 38, suction in inner lumen 42 of inner tubular portion 6 draws tissue into the space between second inner lateral cutting edge 32 and first outer lateral cutting edge 22, where it is trapped by the approaching edges and resected. When inner assembly 4 is rotated within outer assembly 2 in second direction 40, suction in inner lumen 42 of inner tubular portion 6 draws tissue into the space between first inner lateral cutting edge 30 and second outer lateral cutting edge 24, where it is trapped by the approaching edges and resected.

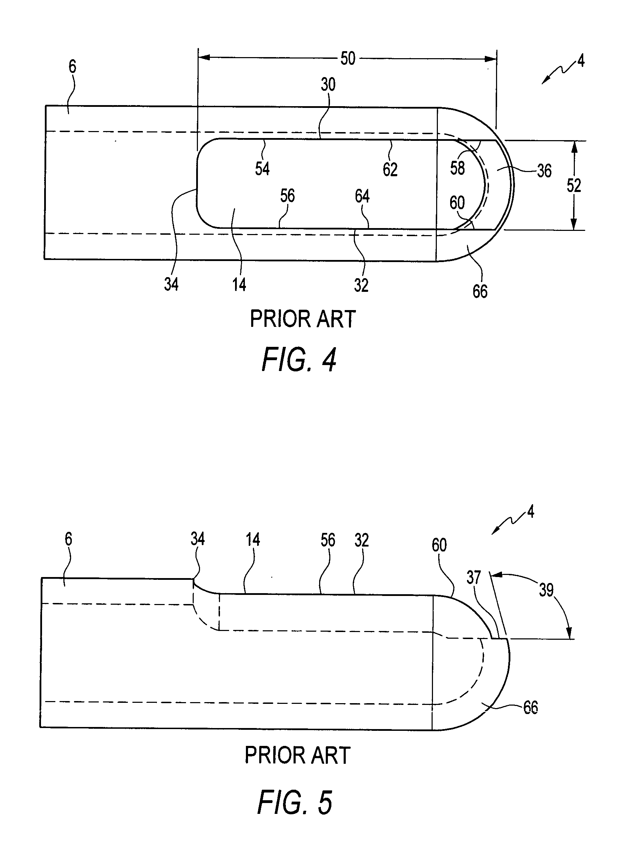

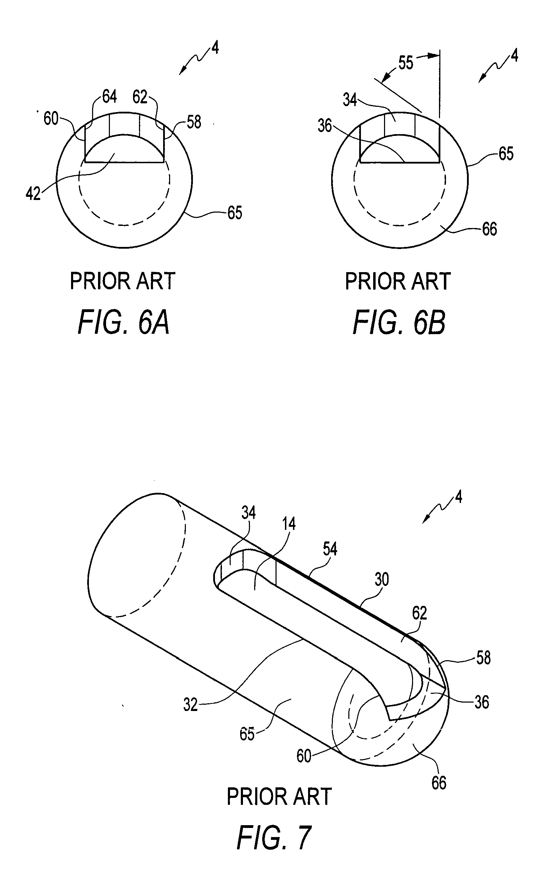

As seen in Figures 4-7, first lateral cutting edge 30 and second lateral cutting edge 32 have a length 50 and are separated by a distance 52. Cutting edges 30 and 32 have linear portions 54 and 56 respectively, and distal curvilinear portions 58 and 60 respectively. Included angle 55 of linear portions 54 and 56 are the angle between tangencies of walls 62 and 64 respectively and cylindrical outer surface 65. Curvilinear portions 58 and 60 are formed by the intersection of first lateral wall 62 and second lateral wall 64 with spherical outer surface 66 of inner tubular portion 6. Distal portion 36 has an inlcued angle 39 formed by surface 37 and a tangent to spherical surface 66. Angle 39 is greater than 90 degrees (obtuse).

Inner window 14 in inner tubular portion 6 can be formed by milling using a conventional (that is "square end") end mill, but is more generally made by EDM using an electrode having a width slightly less than width 52 of window 14, and a length somewhat longer than length 50 of cutting edges 30 and 32. Milling is not the preferred method of manufacture since the endmill diameter must be less than width 52 of window 14. Endmills of these small diameters are prone to breakage and also flexing during use resulting in dimensional variation in the finished parts. The endmills dull quite rapidly during use, further exacerbating the breakage and flexure problems. EDM, however, produces rough surface finishes and irregular cutting edges. Also, the electrodes used to produce the EDMed windows erode during use and must be periodically refurbished. The EDM process is, however, with appropriate fixturing able to produce large numbers of parts in batch operations thereby improving the process economics.

When cutting bone, a shaver is typically used with a constant forward or reverse rotation rather than in an oscillating mode. As described previously, resection of bone is accomplished by the inner cutting edges without cooperative interaction with the outer cutting edges. Linear portions 54 and 56 of edges 30 and 32 have low included angle geometry which will efficiently resect bone. While curvilinear portions 58 and 60 have good geometry in the portions adjacent to linear portions 54 and 56, the portions of the edges adjacent to surface 36 have less cutting efficiency because of interaction between surface 36 and the bone. The distal portion of the cutting edge formed by surface 36 and spherical surface 66 has a large included angle which prevents it from cutting bone. When the portions of the curvilinear edge portions 58 and 60 adjacent to surface 36 penetrate bone, the penetration is limited by interference by surface 36. Prior art shaver 1 is effective for resecting bone when the axis of the tube is substantially parallel to the surface of the bone, so that linear portions 54 and 56 of edges 30 and 32 do most of the resection. Shaver 1 is less effective when the axis of the tube is at a larger angle to the bone surface, so that curvilinear portions 58 and 60 of edges 30 and 32 and surface 36 interact with the bone.

### SUMMARY OF THE INVENTION

Accordingly, it is an object of the present invention to produce an arthroscopic shaver with improved efficiency when cutting bone.

It is also an object of the present invention to produce an arthroscopic shaver which cuts bone efficiently regardless of the portion of the cutting edge in contact with the bone.

It is also an object of the present invention to produce an arthroscopic shaver which efficiently cuts bone and soft tissue.

It is further an object of the present invention to produce a low-cost arthroscopic shaver with improved efficiency when cutting bone.

It is also an object of the present invention to produce a method for making the inner cutting window of an arthroscopic shaver with improved efficiency when cutting bone.

These and other objects are achieved in the present invention by providing an arthroscopic shaver blade with an inner cutting window having two parallel lateral facing edges, a curvilinear distal edge joining the distal ends of the lateral edges, and a curvilinear proximal edge joining the lateral edges. In one embodiment, each lateral cutting edge is formed by the intersection of the cylindrical outer surface of the tubular member and a formed cylindrical surface having an axis approximately parallel to the tube axis. The curvilinear proximal edge is formed by the intersection of the outer cylindrical surface of the tube and a formed spherical surface having a center at the proximal end of the axis of the cylindrical surface which forms the lateral edges, the radius of the formed cylindrical surface and the spherical surface being equal. The curvilinear distal edge is formed by the intersection of the outer distal spherical surface of the tube and a formed spherical surface having a center at the distal end of the axis of the formed cylindrical radius which forms the lateral edges, the radius of the formed cylindrical surface and the formed spherical surface being equal. By varying the axial placement of the formed cylindrical surface forming the distal end of the window, different distal end cutting edge heights can be achieved. For instance, if the formed spherical radius is centered at the center of the tube distal radius, the cutting edge in the distal radius portion of the tube will have a uniform height equal to that of the longitudinal edges. By positioning the center of the spherical radius distal or proximal to the tube distal radius, the edge height in this region may be either respectively decreased or increased.

The window may be formed in a milling operation using a milling cutter having a spherical end (commonly called a "ball-nose" endmill) having an end radius equal to that of the surfaces forming the inner surfaces of the cutting edges.

In another embodiment, a cutting window formed in the manner previously described undergoes a second milling operations using a ball-nose endmill of a smaller diameter. The endmill follows the same path as that of the previous milling operation, however, the endmill is advanced axially (endmill axis) further into the tube to create additional clearance. The cylindrical and spherical surfaces created in this second milling operation intersect the surfaces created in the first milling operation. The resulting cutting edges have a high resistance to plastic deformation because of the larger included angle created by the surfaces of the first milling operation, and are aggressive when cutting soft tissue because of the clearance created by the second milling operation.

In another embodiment, the proximal portion of the window is formed using a ball-nose endmill of a first size, and the distal portion of the window is formed by a ball-nose endmill of a second, smaller size, to create laterally opposed protrusions at the juncture between the proximal and distal portions. These protrusions are positioned approximately at the proximal end of the tube distal radius. The protrusions create areas of high compressive stress when they interact with the surface of bone during use thereby improving cut initiation and enhancing the efficiency of the resection process.

In another embodiment, the lateral edges are formed by the intersection of a series of overlapping spherical surfaces formed in the tube with the outer surface of the tube, the centers of the spherical surfaces being displaced from each other axially (tube axis) so as to form lateral cutting edges having a plurality of teeth. The tooth spacing and included angles are determined by the radius of the spherical pockets, the depth of the pockets in the tube, and the axial spacing between pockets. During use, the teeth create regions of localized high compressive stress when they interact with bone. This aids the initiation portion of the cutting process and enhances the bone cutting efficiency of the shaver. The teeth also aid in preventing soft tissue from being ejected from the window as the cutting edges approach, thereby improving the effectiveness of the resection process. The spherical surfaces are formed using a ball-nose endmill or similar cutter. The cutter is positioned at a first predetermined location and advanced distally in a drilling motion a predetermined distance to form a first spherical surface. The cutter is then withdrawn and repositioned to a second predetermined location and advanced distally to form a second spherical surface. This sequence of metal removal and positioning operations is repeated until all the spherical surfaces are formed.

In another embodiment, the lateral edges are formed by the intersection of a series of overlapping spherical surfaces formed in the tube and the outer surface of the tube, the centers of the spherical surfaces being displaced from each other axially and laterally so as to form lateral cutting edges having a plurality of teeth, the placement of the teeth being asymmetrical about the tube axis when viewed in a plan view. The tooth spacing and included angles of the cutting edges are determined by the radius of the spherical surfaces, the depth of the surfaces in the tube, and the axial and lateral spacing between the spherical surfaces. The function of the teeth is the same as that of the teeth of the previous embodiment when cutting bone and soft tissue. The asymmetric placement of the teeth, however, provides additional benefit when cutting soft tissue since, when used in oscillate mode, the teeth of one direction of rotation interact with tissue portions between those affected by the teeth of the previous, opposite rotation. This is more fully described in copending application 10/937,210, incorporated by reference.

In yet another embodiment, the cutting edges are formed by the intersection of a surface formed by a spherical radius as it traverses a planar nonlinear path, and the outer surface of the tube. The nonlinear path may create, for example, a plurality of teeth, or other shapes to form cutting edges optimized for certain applications.

Other embodiments are made using milling cutters having distal end geometries other than spherical. For instance, distal ends formed to the frustrum of a cone, formed to adjacent conical portions, and formed to geometries having linear and curvilinear portions when viewed in profile are used.

Arthroscopic shavers with inner cutting edges formed in accordance with the principles of this invention are efficient bone cutters because of their low included-angle cutting edges. Because the cutting edges have low included angles in all portions of the cutting window, the shavers are effective resectors of tissue and bone in orientations in which prior art shavers were ineffective. This is particularly true when resecting bone in applications in which resection is accomplished using primarily the distal portion of the cutting edges. Because the cutting edges have low included-angle cutting edges throughtout, they are also efficient tissue cutters. This efficiency is further increased in embodiments having teeth or other protrusions on the cutting edges. The method of forming the window, milling using a cutter with an end formed to a spherical surface, is able to produce cutting edges with high repeatability at low cost. Because the windows are formed by milling, the machined surfaces of the cutting edges have smoother surface finishes and the edges have smaller edge radii than prior art devices which are formed by EDM. This further enhances the efficiency of the shaver when cutting bone or soft tissue.

These and other features and advantages of the invention will be more apparent from the following detailed description that is provided in connection with the accompanying drawings and illustrated exemplary embodiments of the invention.

### BRIEF DESCRIPTION OF THE DRAWINGS

Figure 1 is a disassembled view of a prior art arthroscopic shaver.

Figure 2 is an expanded perspective view of the distal end of the shaver of Figure 1.

Figure 3 is a distal end view of the distal end of the shaver of Figure 2.

Figure 4 is a plan view of the distal end of the inner member of the shaver of Figure 1.

Figure 5 is a side, elevational view of the inner member of Figure 4.

Figures 6A and 6B are an axial distal end views of the inner member of Figure 4.

Figure 7 is a perspective view of the inner member of Figure 4.

Figure 8 is an expanded, perspective view of the distal portion of a shaver constructed in accordance with the principles of the present invention.

Figure 9 is an axial distal end view of the shaver of Figure 8.

Figure 10 is a plan view of the distal portion of the inner member of the shaver of Figure 8.

Figure 11 is a side elevational sectional view of the inner member of Figure 10 at location B - B of Figure 10.

Figure 12 is an axial distal sectional view of the inner member of Figure 10 at location A - A of Figure 10.

Figure 13 is a perspective view of the inner member of Figure 10.

Figure 14 is a plan view of an alternate embodiment of the distal portion of an inner member of a shaver according to the present invention.

Figure 15 is a perspective view of the inner member of Figure 14.

Figure 16 a side elevational sectional view of the inner member of Figure 14 at location A - A Figure 14.

Figures 17A and 17B are distal axial sectional views of the inner member of Figure 14 at location B - B of Figure 14.

Figures 18A and 18B are distal axial sectional views of the inner member of Figure 14 at location C - C of Figure 14.

Figure 19 is a plan view of an alternate embodiment of the distal portion of an inner member of a shaver according to the present invention.

Figure 20 is a side elevational sectional view of the inner member of Figure 19 at location A - A of Figure 19.

Figure 21 is an expanded distal axial sectional view of the inner member of Figure 20 at Location B -B of Figure 19.

Figure 22 is a perspective view of the inner member of Figure 19.

Figure 23 is a plan view of an alternate embodiment of the distal portion of an inner member of a shaver according to the present invention.

Figure 24 is a perspective view of the inner member of Figure 23.

Figure 25 is a side elevational sectional view of the inner member of Figure 23 at location A - A of Figure 23.

Figure 26 is a distal axial sectional view of the inner member of Figure 23 at location B - B of Figure 23.

Figure 27 is a distal axial sectional view of the inner member of Figure 23 at location C - C of Figure 23.

Figure 28 is a plan view of an alternate embodiment of the distal portion of an inner member of a shaver according to the present invention.

Figure 29 is a perspective view of the inner member of Figure 28.

Figure 30A and 30B are expanded distal axial sectional views of the inner member of Figure 28 at location B - B of Figure 28.

Figure 31 is a side elevational sectional view of the inner member of Figure 28 at location A - A of Figure 28.

Figure 32 is a plan view of an alternate embodiment of a shaver inner member constructed in accordance with the principles of the present invention.

Figure 33 is a perspective view of the inner member of Figure 32.

Figure 34 is a side elevational sectional view of the inner member of Figure 32 at location A - A of Figure 32.

Figure 35 is an expanded distal axial sectional view of the inner member of Figure 32 at location B - B of Figure 32.

Figure 36 is a plan view of an alternate embodiment of a shaver inner member constructed in accordance with the principles of the present invention.

Figure 37 is a perspective view of the objects of Figure 36.

Figure 38 is a side elevational sectional view of the objects of Figure 36 at location A - A of Figure 36.

Figure 39 is an axial sectional view of the objects of Figure 36 showing the profile of the distal end of the cutter used to produce the cutting window.

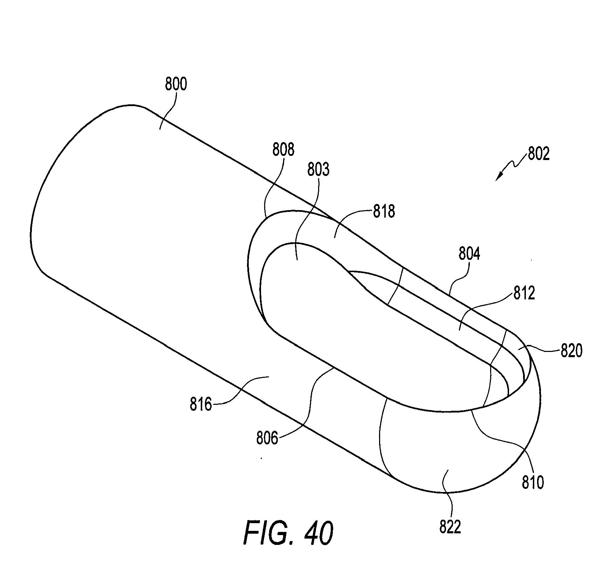

Figure 40 is a perspective view of an alternate embodiment of a shaver inner member constructed in accordance with the principles of the present invention.

Figure 41 is a plan view of the objects of Figure 40.

Figure 42 is a side elevational view of theobjects of Figure 40.

Figure 43 is an axial sectional view of the objects of Figure 40 showing the profile of the distal end of the cutter used to produce the cutting window.

### DETAILED DESCRIPTION OF THE PREFERRED EMBODIMENTS

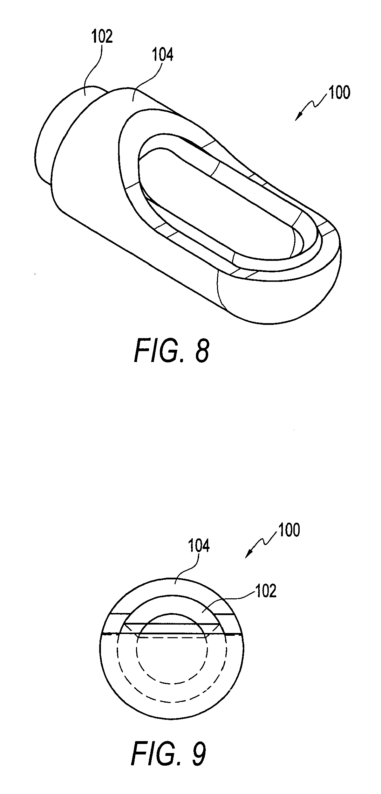

Figures 8 and 9 depict the distal portion of a shaver 100 formed in accordance with the principles of the present invention and having an inner assembly 102 with an inner cutting window, and outer assembly 104 with an outer cutting window. Shaver 100 is operated in a manner identical to that of prior art shaver 1, that is, in oscillate mode, tissue is trapped between opposing inner and outer lateral cutting edges as they approach, first during rotation of the inner assembly in a first direction, and again when the rotation of the inner is reversed. When cutting bone, shaver 100 is used with a constant forward or reverse rotation.

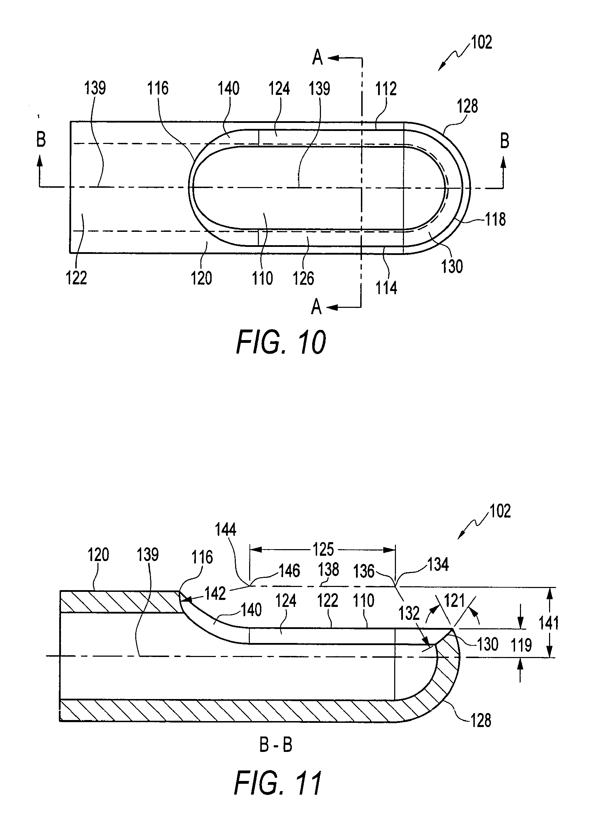

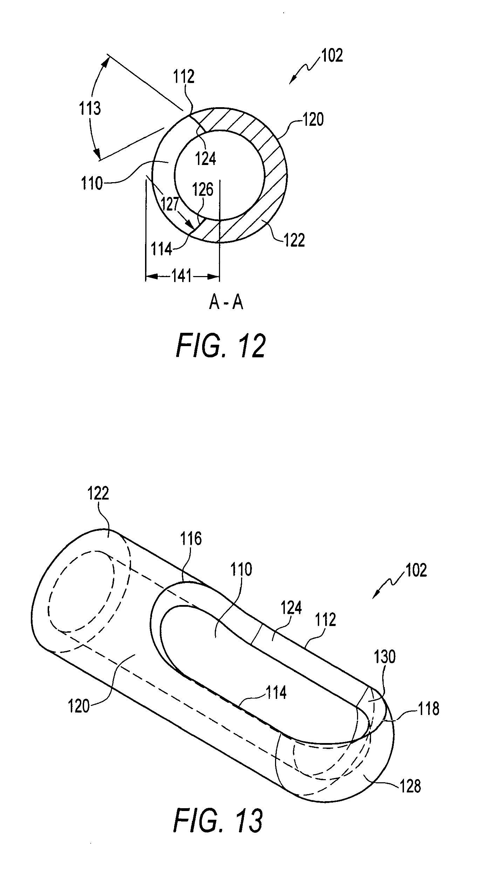

Referring to Figures 10-13, inner cutting window 110 has a first lateral cutting edge 112 and a second lateral cutting edge 114 joined by a curvilinear proximal edge 116 and a curvilinear distal edge 118. As seen in Figure 11 (showing a side elevational sectional view in direction B - B (Figure 10)) and in Figure 12 (showing an axial sectional view in direction A - A (Figure 10)), lateral cutting edges 112 and 114 have low included angles throughout, the edges being formed by the intersection of the cylindrical outer surface 120 of the tubular member 122 and cylindrical surfaces 124 of first lateral edge 112 and 126 of second lateral edge 114, cylindrical surfaces 124 and 126 of length 125 being approximately coaxial, and of radius 127. The included angle 113 of first lateral cutting edge 112 is defined as the angle between the tangencies of outer surface 120 and surface 124 at the cutting edge; the included angle of second lateral edge 114 is defined as the angle between the tangents of outer surface 120 and cylindrical surface 126 and is equal to angle 113. Angle 113 is preferably between 20 and 85 degrees, and more preferably between 30 and 70 degrees.

Curvilinear distal edge 118 has a low included angle throughout, edge 118 being formed by the intersection of spherical outer surface 128 of tubular member 122, and spherical surface 130 of radius 132 equal to radius 127. The included angle 119 of distal edge 118 is defined as the angle between the tangents of spherical outer surface 128 and spherical surface 130 at any point on edge 118. Angle 119 is preferably between 20 and 75 degrees, and more preferably between 30 and 70 degrees.

Center 134 of spherical surface 130 is at distal end 136 of axis 138 of cylindrical surfaces 124 and 126. Axis 138 is parallel to axis 139 of tubular member 122 and displaced therefrom a distance 141. Curvilinear proximal edge 116 is formed by the intersection of cylindrical outer surface 120 of tubular member 122, and spherical surface 140 of radius 142 equal to radii 127 and 132. Center 144 of spherical surface 140 is at proximal end 146 of axis 138 of cylindrical surfaces 124 and 126. When viewed in a plan view as in Figure 10, distal spherical surface 130 of edge 118 is centered within distal spherical surface 128 of outer tube 122 so that distal edge 118 is of constant height 119, as seen in Figures 11 and 12. In other embodiments, when viewed in plan view the center of inner spherical surface 130 is distal to the center of outer spherical surface 128 so that the distal portion of cutting edge 118 is not of constant height, but rather has a lower height at its distal end. In other embodiments inner surface 130 is proximal to the center of surface 128 so as to make the distal portion of edge 118 higher than the more proximal portions of the cutting window.

A preferred method for forming inner cutting window 110 is by milling with an endmill having a spherical, distal end cutting surface (commonly called a "ball nose" end mill), the radius of the spherical tip being equal to radii 127, 132, and 142. The endmill is oriented with its axis perpendicular to the tube axis, centered with the tube axis and the endmill is advanced axially to machine into tube 122 until the center of the spherical radius is distance 141 from axis 139 of tube 122. The endmill is then fed linearly parallel to axis 139 of tube 122 distance 125, the distance between the centers of spherical distal surface 130 and spherical proximal surface 140.

Because the window is formed by a portion of the distal radius of an endmill with a spherical end, the endmill diameter can be larger than that used to form the cutting window of the prior art device. This results in less breakage of the endmill and decreased flexing of the endmill during machining of the window. This, in turn, allows the use of more aggressive feed rates, decreases the cycle times and makes forming of the window more economical.

Because distal edge 118 has a low included angle throughout, inner window 110 is able to resect bone more effectively than prior art shavers when the angle between the axis 139 of inner tube 122 and the bone surface is such that contact between the bone and cutting window 110 occurs primarily in the distal portion of the cutting edge.

Cutting edges 112,114 and 118 of window 110 have a uniform height throughout. Creating protrusions or irregularities on the cutting edges creates areas of localized higher compressive stress when resecting bone thereby improving the cut initiation and resection efficiency. Accordingly, other embodiments have protrusions or teeth which aid in resecting bone.

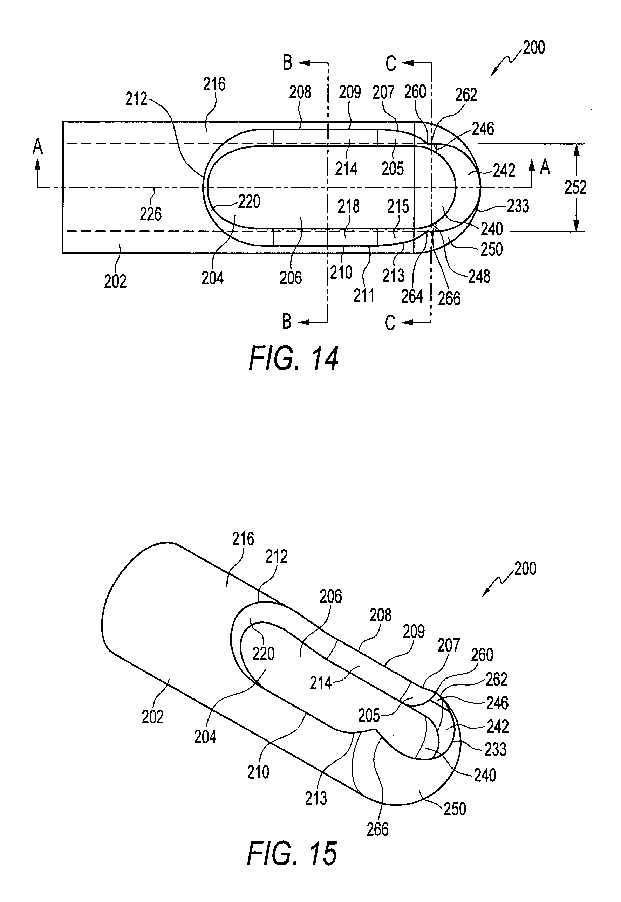

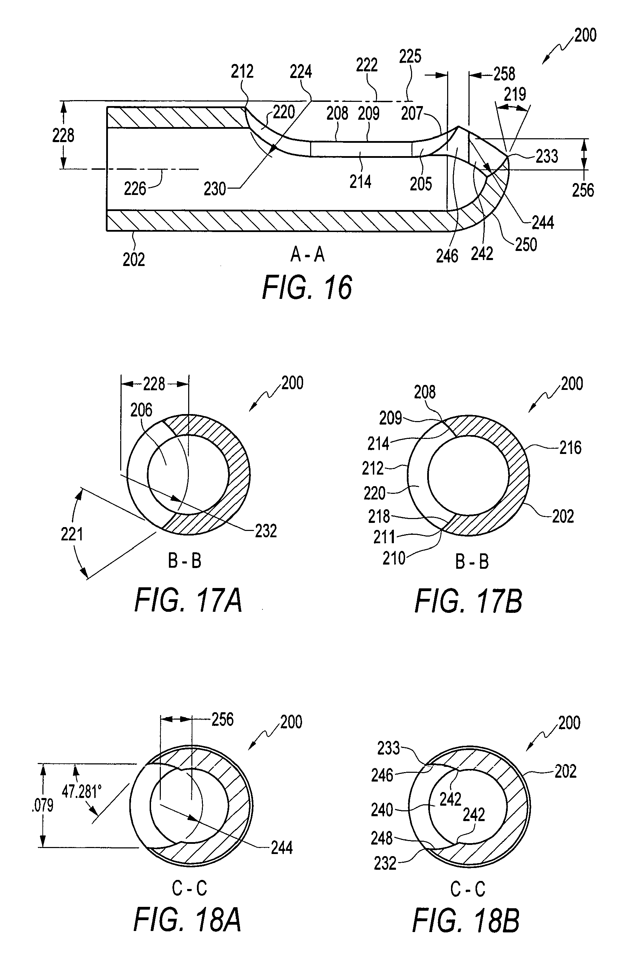

Referring now to Figures 14 -18 showing the distal portion 200 of the inner tubular member 202 of an alternate embodiment, cutting window 204 has a first or proximal portion 206 having a first cutting edge 208 and a second cutting edge 210 joined by proximal edge 212. Linear portion 209 of edge 208 is formed by the intersection of cylindrical surface 214 and outer cylindrical surface 216 of tubular member 202. Linear portion 211 of edge 210 is formed by the intersection of cylindrical surface 218 with outer cylindrical surface 216 of tubular member 202. Cylindrical surfaces 214 and 218 have a common axis 222. Included angle 221 of linear portion 211 of edge 210 is defined as the angle between the tangencies of cylindrical surfaces 218 and 216. The included angle of linear portion 209 of edge 208 is defined as the angle between the tangencies of cylindrical surfaces 214 and 216, and is equal to angle 221. Angle 221 is preferably between 20 and 85 degrees, and more preferably between 30 and 70 degrees.

Curvilinear portion 207 of edge 208 is formed by the intersection of spherical surface 205 and outer cylindrical surface 216 of tubular member 202. Curvilinear portion 213 of edge 210 is formed by the intersection of spherical surface 215 and outer cylindrical surface 216 of tubular member 202. Proximal edge 212 is formed by the intersection of spherical surface 220 with outer surface 216 of tubular member 202. Center 224 of spherical surface 220 is at the proximal end of axis 222 of cylindrical surfaces 214 and 218. Center 225 of spherical surfaces 205 and 215 is at the distal end of axis 222. Axis 222 is parallel to axis 226 of tubular member 202 and displaced from axis 226 a distance 228. Radius 232 of cylindrical surfaces 214 and 218 is equal to radius 230 of spherical surfaces 205, 215 and 220.

Second or distal portion 240 of window 204 has a curvilinear cutting edge 233 formed by the intersection of spherical surface 242 of radius 244 and laterally opposed parallel planar surfaces 246 and 248 with outer spherical surface 250 of tubular member 202. Distance 252 between surfaces 246 and 248 is equal to twice radius 244. Center 254 of spherical surface 242 is displaced a distance 256 from axis 226 of tubular member 202, and is displaced axially a distance 258 distal to the center of spherical outer surface 250 of tube 202.

Window 204 is formed using a two-step milling process. Proximal portion 206 is formed using a ball-nose end mill having a spherical radius equal to radius 232 of cylindrical surfaces 214 and 218 and spherical radius 230 of spherical surfaces 205, 215 and 220 in the same manner as inner cutting window 110 (Figures 10-13). Distal portion 240 of window 204 is formed using a ball nose endmill having a spherical radius equal to radius 244 of spherical surface 242. Proximal portion 206 is machined first using the larger diameter endmill to minimize the amount of material which is removed using the smaller diameter endmill to form distal portion 240 of window 204. In this way, the metal removal rate during machining of the window is maximized and the machining cost minimized.

Window 204 has a first protrusion (or tooth) 260 formed by curvilinear portion 207 of first edge 208 of proximal window portion 206, and first proximal edge portion 262 of cutting edge 233 of distal portion 240 of window 206. Window 204 also has a second protrusion (or tooth) 264 formed by curvilinear portion 213 of second edge 210 of proximal window portion 206, and second proximal edge portion 266 of cutting edge 233 of distal portion 240 of window 206. Protrusions 260 and 264 have low included angles and act as teeth which readily penetrate soft tissue so as to aid in preventing soft tissue from being ejected as inner and outer cutting edges approach each other during use. Protrusions 260 and 264 also create areas of localized high compressive stress in bone when resecting bone so as to aid in the initiation of resection. The distal placement of the protrusions allows the shaver to resect bone more effectively than prior art shavers when the angle between the axis 226 of inner tube 202 and the bone surface is such that contact between the bone surface and cutting window 204 occurs primarily in the distal portion of the cutting edge. All cutting edges of window 204 have low included angles throughout.

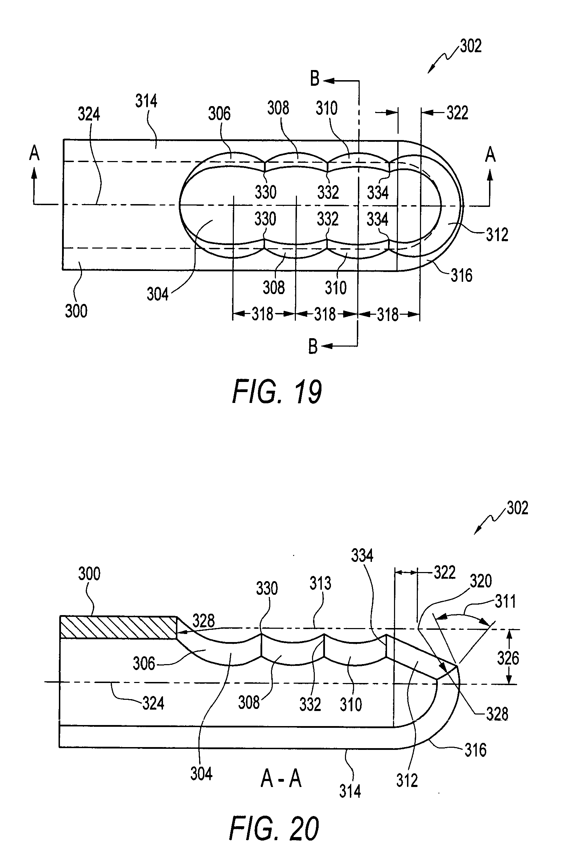

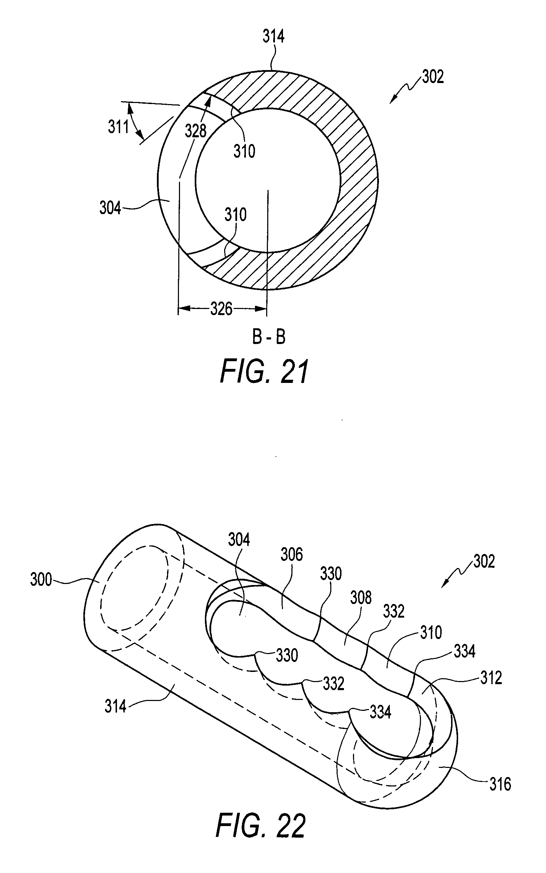

An embodiment having multiple protrusions for enhanced efficiency when cutting tissue or bone is depicted in Figures 19-22. Distal end 302 of inner tube 300 has a cutting window 304 formed by the intersection of spherical surfaces 306, 308, 310 and 312 with cylindrical outer surface 314 and spherical distal surface 316 of tube 300. The centers of spherical surfaces 306 through 312 are separated axially by distance 318. Center 320 of surface 312 is displaced distally from the center of spherical distal surface 316 of tube 300 by distance 322. The centers of spherical surfaces 306 through 312 lie on a line 313 which is parallel to axis 324 of tube 300 and displaced a distance 326 therefrom. Spherical surfaces 306 through 312 have a common radius 328. The intersection of spherical surfaces 306 and 308 form teeth 330, the intersection of radii 308 and 310 form teeth 332, and the intersection of radii 310 and 312 form teeth 334. Cutting window 304 has low included angles 311 throughout, the angles being defined as the angle between the tangencies of cylindrical surfaces 306, 308, 310, 312 and cylindrical outer surface 314 or spherical distal surface 316. Angle 311 is preferably between 20 and 85 degrees, and more preferably between 30 and 70 degrees.

Window 304 is formed by a four-step operation using a ball-nose endmill having a spherical radius equal to radius 328 of spherical surfaces 306 through 312. The endmill is positioned with its axis perpendicular to axis 324 of tube 300 and positioned a distance 322 distal from the center of spherical surface 316 of tube 300. While rotating, the endmill is advanced distally along its axis until the center of the end mill spherical radius is a distance 326 from axis 324 of tube 300 to form spherical surface 312. The endmill is retracted and repositioned by moving proximally distance 318 along tube axis 324. The endmill is advanced distally along its axis until the center of the endmill spherical radius is distance 326 from axis 324 of tube 300 to form spherical surface 310. This sequence of repositioning and machining operations is repeated so as to form spherical radii 308 and 306. Because the spherical surfaces are formed by the spherical end of a ball-nose endmill, the diameter of the endmill is greater than that of endmills necessary to produce prior art shaver inners. Additionally, because the endmill forms the spherical surfaces through a drilling (axially advancing) motion, wear of the end mill cutting edges occurs across the entire portion of the endmill spherical radius which forms the pocket, rather than being concentrated in the portion which intersects the tube wall when an endmill is moved laterally along a predetermined path. This minimizes localized wear which affects product surface quality and consistency.

As with the previous embodiment illustrated in Figures 14-18 above, the protrusions (teeth) on the cutting edges minimize the ejection of soft tissue from the cutting windows as the cutting edges approach each other. Also, when cutting bone, the teeth produce locally concentrated areas of high compressive stress when the teeth contact a bone surface. This aids the initiation of resection and enhances the efficiency of the removal process. All cutting edges of this embodiment have low included angles throughout, preferably between 20 and 85 degrees, and more preferably between 30 and 70 degrees.

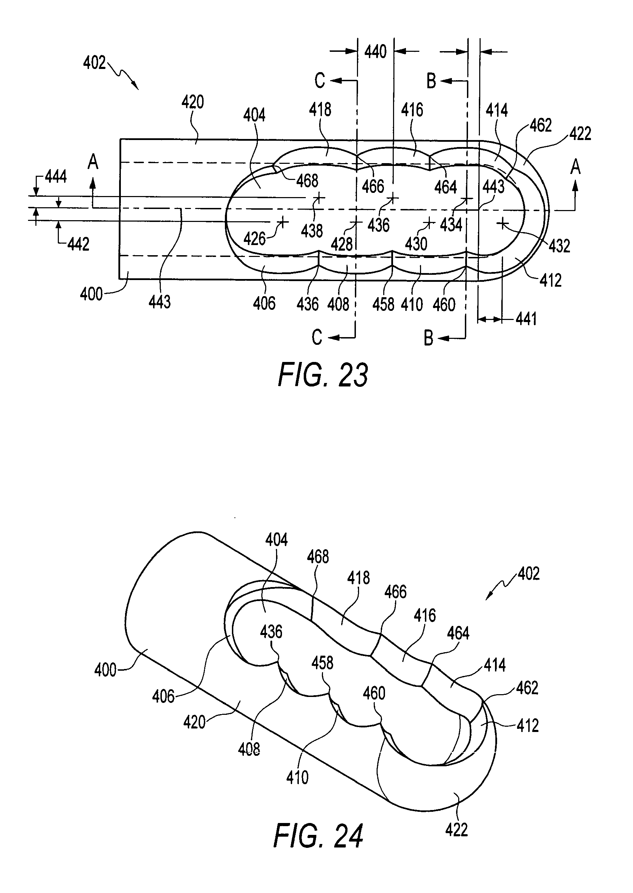

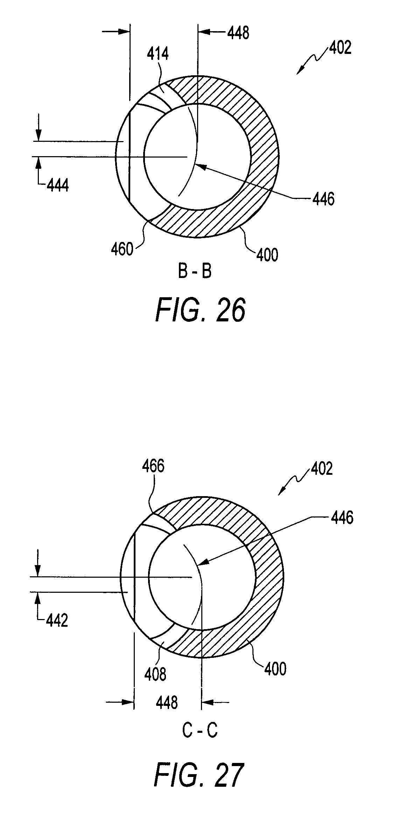

In yet another embodiment shown in Figures 23-27, distal end 402 of inner tube 400 has a cutting window 404 formed by the intersection of spherical surfaces 406 through 418 with cylindrical outer surface 420 and spherical surface 422 of tube 400. When viewed in a plan view as in Figure 23, centers 426 through 438 of spherical surfaces 406 through 418, respectively, are spaced axially a distance 440. Center 432 of spherical surface 412 is displaced axially distance 441 distal to center 443 of spherical surface 422 of tube 400. Centers 426, 428, 430 and 432 of spherical surfaces 406, 408, 410 and 412 are laterally offset in a first direction a distance 442 from axis 443 of tube 400. Centers 434, 436 and 438 of spherical surfaces 414, 416 and 418 are laterally offset in a second direction a distance 444. Spherical surfaces 406 through 418 have a common radius 446.

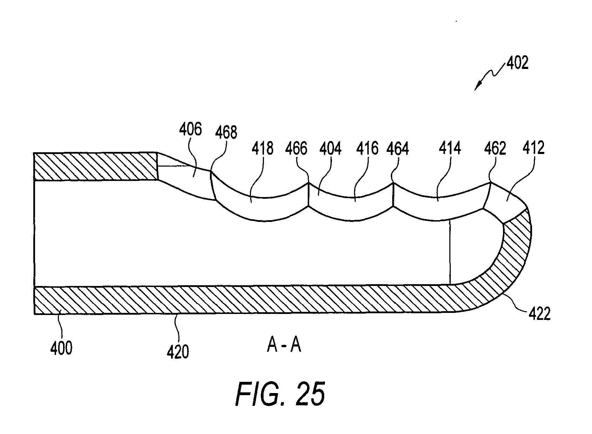

As illustrated in Figures 25-27, the plane containing centers 426 through 438 is displaced a distance 448 from axis 443 of tube 400. Tooth 456 is formed by the intersection of spherical surfaces 406 and 408; tooth 458 is formed by the intersection of spherical surfaces 408 and 410; tooth 460 is formed by the intersection of spherical surfaces 410 and 412; tooth 462 is formed by the intersection of spherical surfaces 412 and 414; tooth 464 is formed by the intersection of spherical surfaces 414 and 416; tooth 466 is formed by the intersection of spherical surfaces 416 and 418. All cutting edges of window 404 have low included angles throughout, the angles being defined as the angle between the tangencies of spherical surfaces 406 through 418 and cylindrical outer surface 420 or spherical outer surface 422 at any point on the cutting edges. The included angles are preferably between 20 and 85 degrees, and more preferably between 30 and 70 degrees.

Window 404 is formed by a multi-step operation using a ball-nose endmill in the same manner as the previous embodiment shown in Figures 19-22, using a ball-nose endmill with a spherical radius equal to radius 446 of spherical surfaces 406 through 418. The endmill is positioned with its axis perpendicular to axis 443 of tube 400 and positioned a distance 441 distal from the center of spherical surface 422 of tube 400 and a distance 442 laterally in a first direction from axis 443. While rotating, the endmill is advanced distally until the center of the endmill spherical radius is a distance 448 from the plane normal to the cutter axis containing axis 443 of tube 400 so as to form spherical surface 412. The endmill is retracted and repositioned to center 434 of spherical surface 414. The endmill is advanced distally to form spherical surface 414. This sequence of repositioning and machining operations is repeated to form spherical surfaces 410, 416, 408, 418, and 406. Because the spherical surfaces are formed by the spherical end of a ball-nose end mill, the diameter of the endmill can be greater than that of end mills necessary to produce prior art shaver inners. Additionally, because the endmill forms the spherical surfaces through a drilling (axially advancing) motion, wear of the end mill cutting edges occurs across the entire portion of the endmill spherical radius which forms the pocket, rather than being concentrated in the portion which intersects the tube wall when an endmill is moved laterally along a predetermined path. This minimizes localized wear which affects product surface quality and consistency.

As with the previous embodiment shown in Figures 19-22 above, the protrusions (teeth) on the cutting edges minimize the ejection of soft tissue from the cutting windows as the cutting edges approach each other. The asymmetric positioning of the teeth when viewed in a plan view also enhances the efficiency of the shaver when used on tissue in an oscillate mode. When the inner member is rotated in a first direction, the teeth on a first side of the cutting window engage tissue, the teeth preferentially removing tissue in tissue regions which the teeth engage. When the inner rotation is reversed, the teeth of the opposite cutting edge engage regions which were not affected by the teeth of the previous rotation direction. This results in reduced ejection of tissue from the cutting window as the cutting edges approach each other. Also, when cutting bone, the teeth produce locally concentrated areas of high compressive stress in bone when the teeth contact a bone surface. This helps the initiation of resection and enhances the efficiency of the removal process.

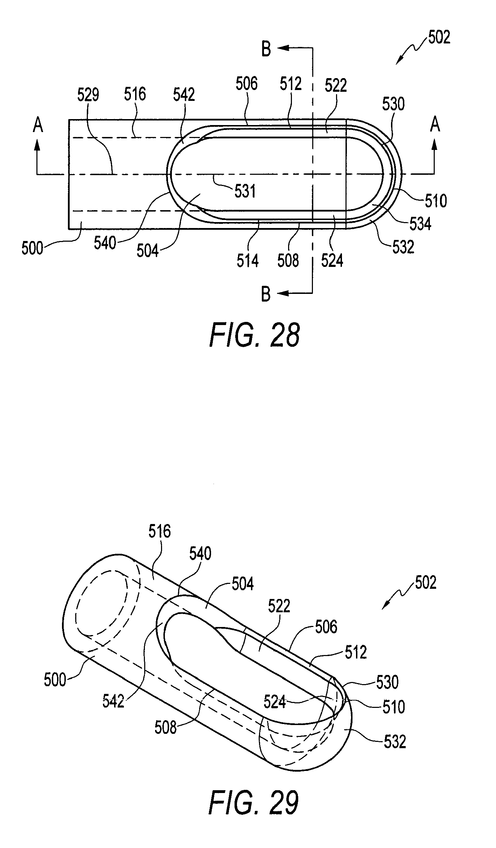

In certain circumstances, it is desirable to have cutting edges with high strength for cutting bone, and also low included angles for aggressive resection of soft tissue. This is accomplished in the embodiment shown in Figures 28-31. The inner member is a modification of the shaver inner member 102 shown in Figures 10-13. Specifically, a portion of the cylindrical surfaces (which in combination with tube outer surface form the lateral linear cutting edges) and a portion of the spherical surface (which in combination with the outer spherical surface of the inner tube form the distal curvilinear cutting edge) are removed to facilitate the flow of tissue into the inner lumen for resection and aspiration from the site.

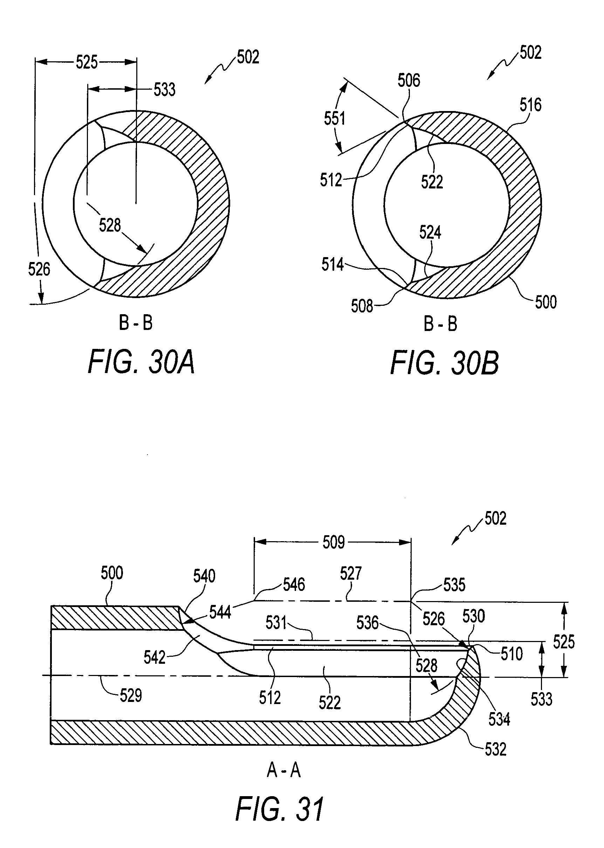

Referring now to Figures 28-31, distal portion 502 of inner tube 500 has a cutting window 504. First lateral cutting edge 506 and second lateral cutting edge 508 of length 509 are formed by the intersections of first cylindrical surfaces 512 and 514 with cylindrical surface 516 of inner tube 500. First cylindrical surfaces 512 and 514 extend from cutting edges 506 and 508 to second cylindrical surfaces 522 and 524 respectively. First cylindrical surfaces 512 and 514 are coaxial, have a radius 526 and have an axis 527 displaced distance 525 from axis 529 of tube 500. Second cylindrical surfaces 522 and 524 are coaxial, have a radius 528, and an axis 531 displaced distances 533 from axis 529 of tube 500. Distal cutting edge 510 is formed by the intersection of first spherical surface 530 with spherical surface 532 of tube 500. First spherical surface 530 extends from cutting edge 510 to second spherical surface 534. Center 535 of first spherical surface 530 is at the distal end of axis 527 of first cylindrical surfaces 512 and 514. Center 536 of second spherical surface 534 is at the distal end of axis 531 of second cylindrical surfaces 522 and 524. Proximal edge 540 of window 504 is formed by the intersection of spherical surface 542 with outer cylindrical surface 516. Spherical surface 542 of radius 544 has a center 546 at the proximal end of axis 527 of first and second cylindrical surfaces 512 and 514. As seen in Figure 30B, included angle 551 is defined as the angle between the tangencies of cylindrical surfaces 512 and 514 and outer cylindrical surface 516, and is equal to the included angle of distal cutting edge 510 which is defined as the angle between the tangencies of cylindrical surface 530 and spherical surface 532 at any point on edge 510. Angle 551 is preferably between 20 and 85 degrees, and more preferably between 30 and 70 degrees.

Window 504 is formed in a two-step milling operation. A first operation forms first cylindrical surfaces 512 and 514, and spherical surface 530 in a milling operation identical to that used to form window 110 of the previous embodiment shown in Figures 10-13. Second cylindrical surfaces 522 and 524, and spherical surface 534 are formed in a second milling operation using a ball-nose endmill with a spherical radius equal to radius 528 of spherical surface 534. The endmill is positioned with its axis perpendicular to axis 529 of tube 500, and centered with spherical surface 534. While rotating, the endmill is advanced until the center of the endmill radius is a distance 533 from tube radius 529. The endmill is then fed proximally parallel to the tube axis 529 distance 509, and retracted at the completion of the motion.

The cutting edges of window 504 have a higher resistance to plastic deformation than the edges of window 110 depicted inFigures 10 through 13 because of the larger included-angle, and increased aggressiveness when cutting soft tissue due to the low included angle portions of the cutting edges formed by second cylindrical surfaces 522 and 524, and by second spherical surface 534. The cutting edges of this embodiment, while more resistant to edge deformation when cutting bone, are able to penetrate tissue efficiently because of the decreased included angle of portions of the cutting edge formed by cylindrical surfaces 522 and 524 and spherical surface 534.. All cutting edges have low included angles throughout, preferably between 20 and 85 degrees, and more preferably between 30 and 70 degrees.

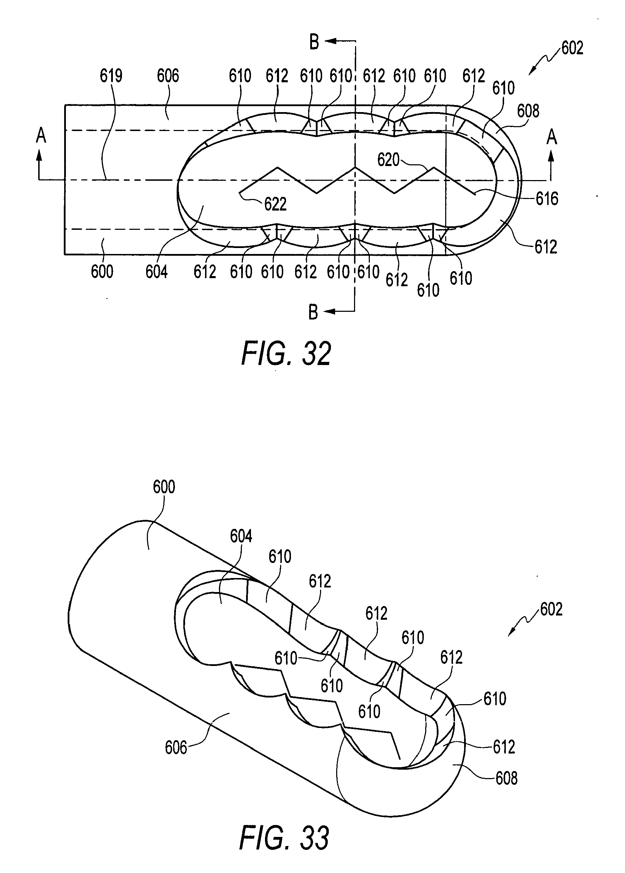

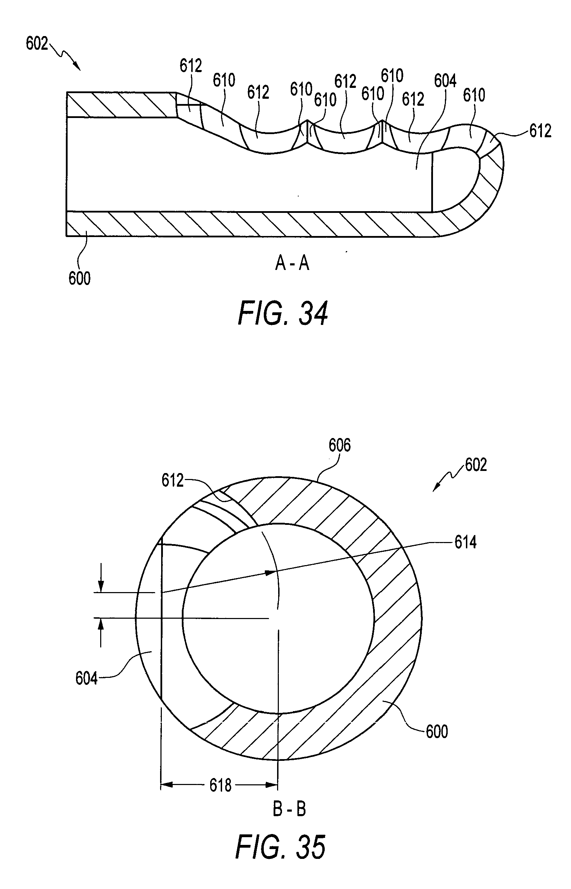

In yet another embodiment shown in Figures 32-35 depicting the distal end 602 of inner tubular member 600 with a cutting window 604 formed therein, window 604 is formed by the intersection of outer cylindrical surface 606 and spherical surface 608 of tube 600 with cylindrical surfaces 610 and spherical surfaces 612 formed in tube 600. The included angles of the cutting edges is low throughout, including in the far distal portion of the window. The included angles may be varied by changing the size of radius 614, height 618, and path 616. The included angles are preferably between 20 and 85 degrees, and more preferably between 30 and 70 degrees.

Cylindrical surfaces 610 and spherical surfaces 612 are formed by a milling cutter having a spherical cutting surface formed at its distal end, the radius of the distal end being equal to radius 614 of surfaces 610 and 612. The milling cutter is advanced distally into tube 600 at first location 616 until the center of the cutter distal radius is a distance 618 above the plane normal to the cutter axis containing axis 619 of tube 600. The cutter is then moved along cutter path 620 to location 622 and retracted. This embodiment is used in the same manner as the previous embodiments, the teeth having the same function as the teeth in the previous embodiments, that is, to prevent the ejection of tissue from the window when cutting soft tissue, and to create areas of high compressive stress at the bone surface when cutting bone so as to increase cut initiation efficiency. The shape of window 604 is determined by cutter path 620, the configuration of the milling cutter, and distance 618. By varying these parameters, the number of teeth may be increased or decreased, the cutting edges may have larger or smaller included angles, or edge configurations with irregular contours may be created as specific applications require.

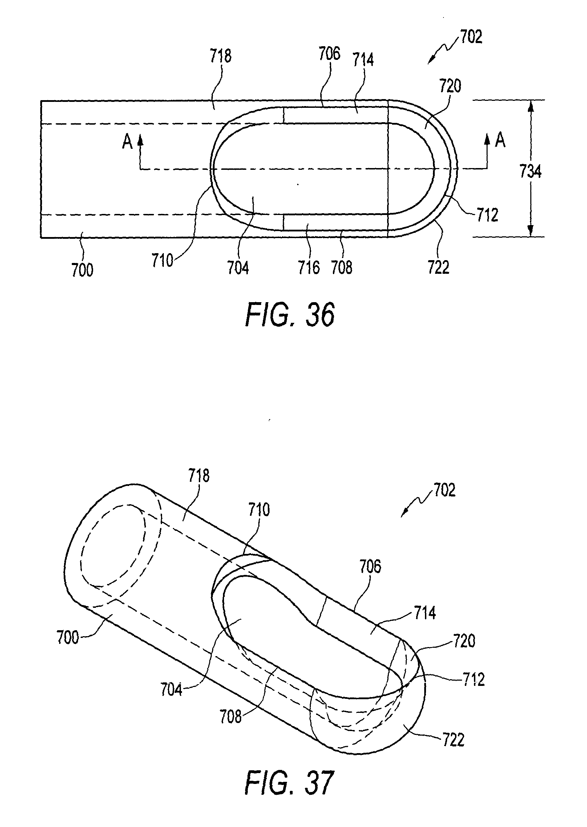

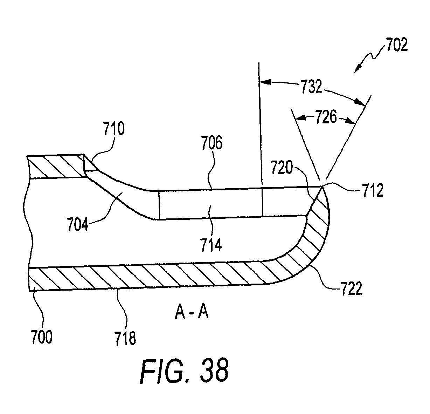

Embodiments previously herein described have been formed using milling cutters having their distal ends formed to a radius. Other embodiments are formed using milling cutters having distal ends formed to other geometries. For instance, Figures 36 through 39 depict the distal end 702 of a shaver inner tubular member having a cutting window 704 formed by milling using a cutter having a distal end formed to the frustrum of a cone. Cutting window 704 has a first lateral cutting edge 706 and a second lateral cutting edge 708, a proximal edge 710 connecting edges 706 and 708, and a distal edge 712 connecting the distal ends of edges 706 and 708. Lateral edges 706 and 708 are formed by the intersection of planar surfaces 714 and 716 respectively with tube cylindrical outer surface 718. Distal cutting edge 712 is formed by the intersection of conical surface 720 and tube spherical surface 722. Lateral edges 706 and 708, have an included angle 724 defined as the angle between the tangencies to cylindrical surface 718 and surfaces 714 and 716 respectively. Distal edge 712 has an included angle 726 defined as the angle between a tangency to spherical surface 722 and conical surface 720 at any point on edge 712. Angles 724 and 726 are preferably between 20 and 85 degrees, and more preferably between 30 and 70 degrees.

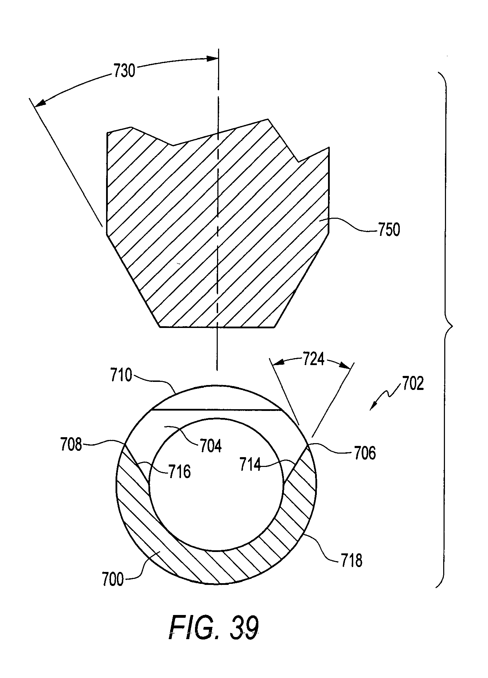

The milling cutter used to produce window 704, the profile of the distal-most portion of which is depicted in Figure 39, has a distal end 750 forming the frustrum of a cone having an included angle 730 so as to form surfaces 714 and 716 to the same included angle. Angle 732 of conical surface 720 is equal to half of included angle 730 of the conical portion of cutter distal end 750. Window 704 is formed in the same manner as the previous embodiments, that is, cutter 750 is positioned at a first location centered above distal surface 722. While rotating, cutter 730 is advanced into tubular member 500 to a predetermined depth. When the depth is reached, the cutter is fed axially (tube axis) until lateral edges 706 and 708 are the desired length. The cutter is then withdrawn.

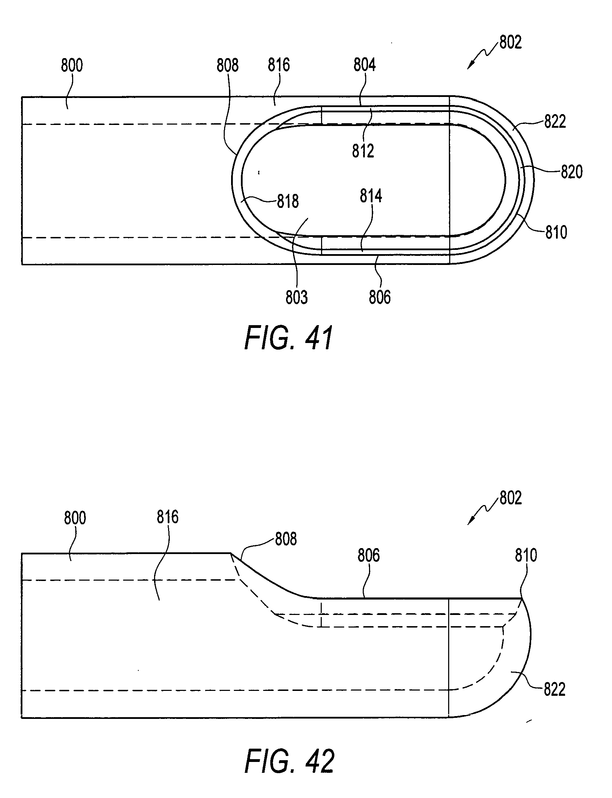

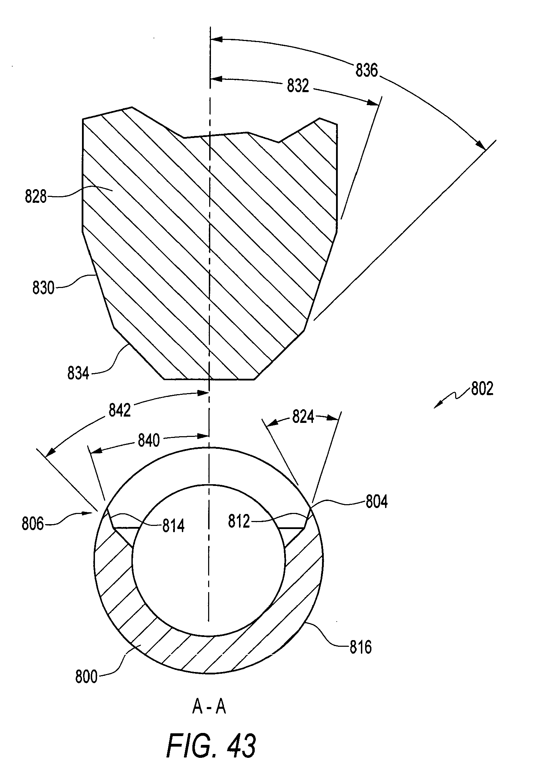

In yet another embodiment depicted in Figures 41 through 43, the window is formed by a milling cutter having a distal portion formed of adjacent conical portions, the distal conical portion having a larger included angle than the proximal conical portion. The window formed has a decreased included angle at the cutting edges so as to provide enhanced performance when cutting tissue, and improved efficiency when resecting bone since the decreased included angle increases the compressive stress at the bone surface for improved cut initiation. Distal portion 802 of shaver tubular inner member 800 has a window 803 having a first lateral edge 804, a second lateral edge 806, a curvilinear proximal edge 808 and a curvilinear distal edge 810. First lateral edge 804 and second lateral edge 806 are formed by the intersection of first planar surface 812 and second planar surface 814 respectively with outer cylindrical surface 816 of tubular member 800. Curvilinear proximal edge 808 is formed by the intersection of conical surface 818 with outer surface 816. Curvilinear distal edge 810 is formed by the intersection of conical surface 820 with spherical outer surface 822. Included angle 824 of first lateral edge 804 is defined as the angle between first planar surface 812 and a tangent to cylindrical surface 816. The included angle of second lateral edge 806 is similarly defined and is equal to angle 824. The included angle of curvilinear distal edge 810 is defined as the angle between conical surface 820 and a tangent to spherical outer surface 822 at any point on edge 810 and is equal to angle 824. The milling cutter used to produce window 804, the profile 828 of the distal portion of which is depicted in Figure 43, has a proximal conical portion 830 with an included angle 832 and a distal conical portion 834 with an included angle 836. Included angle 832 of proximal portion 830 determines the angle of inclination 840 of planar surfaces 812 and 814 and conical surface 820 which is half of angle 832. Included angle 836 of distal conical portion 834 determines the angle of inclination 842 of second planar surfaces 844 and 846 and second conical surface 848. Milling of window 803 is accomplished in the same manner as previous embodiment 702 shown in Figures 36 through 39.

The included angles of cutting edges produced in accordance with the principles of this invention are determined by the configuration of the distal end of the milling cutter used to produce the window, and the characteristics of the millng process. The distal end of the milling cutter used to produce all embodiments herein described, both with and without teeth, may be spherical, elliptical, conical, or have a complex geometry wherein the profile when viewed in section is comprised of linear and curvilinear segments. The milling processes as described herein may be accomplished in a single operation, or may comprise roughing and finishing passes to produce the finished geometry.

The cylindrical machined surfaces formed in the embodiments described above are coaxial and have axes parallel to the tube axis. In certain applications, however, it may be advantageous for the cylindrical surfaces to have axes that are not parallel to the tube axis, or to have machined cylindrical surfaces which are not coaxial. Such configurations are anticipated and within the scope of this invention.

The above description and drawings illustrate preferred embodiments which achieve the objects, features and advantages of the present invention. It is not intended that the present invention be limited to the illustrated embodiments. Any modification of the present invention which comes within the spirit and scope of the following claims should be considered part of the present invention.

## Claims

1. A surgical shaver instrument for cutting anatomical tissue, comprising:
an outer tubular member having a distal opening at the distal end for receiving anatomical tissue therethrough;
an inner tubular member having an inner cylindrical surface, an outer cylindrical surface, a distal end, a proximal end, a longitudinal axis, and a distal opening at the distal end for receiving anatomical tissue therethrough, wherein the distal opening further comprises a first lateral edge, a second lateral edge, a curvilinear proximal edge in contact with the first and second lateral edges, a curvilinear distal edge in contact with the first and second lateral edges, and wherein the first lateral edge, the second lateral edge, and the curvilinear distal edge having included angles which are acute.

2. The surgical shaver instrument of claim 1, wherein the included angles are between 20 and 85 degrees.

3. The surgical shaver instrument of claim 1, wherein the included angles are between 30 and 70 degrees.

4. The surgical shaver instrument of claim 1, wherein the first lateral edge comprises at least one tooth, and the second lateral edge comprises at least one tooth.

5. The surgical shaver instrument of claim 4, wherein the at least one tooth of the first lateral edge and the at least one tooth of the second lateral edge are asymmetrically located relative to a longitudinal axis of the tubular member.

6. The surgical shaver instrument of claim 4, wherein the at least one tooth of the first lateral edge and the at least one tooth of the second lateral edge are symmetrically located relative to a longitudinal axis of the tubular member.

7. A method of manufacturing the inner tubular member of the surgical shaver instrument of claim 1, comprising:
providing a tubular member having an axis, a proximal end and a closed distal end;
providing a first milling cutter having a distal end formed of a first predetermined shape;
positioning the distal end of the tubular member at a first location relative to the first milling cutter; and
conducting at least one milling process employing the first milling cutter to form a cutting region on the distal end of the tubular member, the cutting region comprising a first lateral cutting edge, a second lateral cutting edge, a curvilinear proximal edge in contact with the first and second lateral cutting edges, and a curvilinear distal edge in contact with the first and second lateral cutting edges;
wherein the first lateral edge, second lateral edge and curvilinear distal edge have included angles which are acute.

8. The method of claim 7, wherein the step of conducting the at least one milling process further includes the step of conducting a second milling process with a second milling cutter having a distal end formed to a second predetermined shape, subsequent to the step of conducting the at least one milling process.

9. The method of claim 8, wherein the diameter of the second milling cutter is different from the diameter of the first milling cutter.

10. The method of claim 7, wherein the configuration of the distal end of the milling cutter is spherical.

11. The method of claim 7, wherein the configuration of the distal end of the milling cutter comprises at least one frustum of a cone.

12. The method of claim 7, wherein the configuration of the distal end of the milling cutter is curvilinear when viewed in profile.

13. The method of claim 7, wherein the configuration of the distal end of the milling cutter is a collection of linear and curvilinear segments when viewed in profile.
